# EUROPÄISCHE PATENTSCHRIFT

(11) **EP 0 271 785 B1**
(45) Veröffentlichungstag und Bekanntmachung des Hinweises auf die Patenterteilung: **10.03.1993**
(21) Anmeldenummer: 87117993.3
(22) Anmeldetag: 04.12.1987
(51) Int. Cl.: A61M 5/14

(54) **Dosiereinrichtung zur Einstellung der Durchflussrate einer Flüssigkeitsströmung in den oder aus dem Körper eines Lebewesens**
Dosing device for setting the liquid flow into or out of a living body
Dispositif de dosage du débit d'un écoulement de liquide dans le corps d'un être vivant

(30) Priorität: 15.12.1986 DE 3642812; 09.01.1987 DE 3700502; 20.08.1987 DE 3727816
(43) Veröffentlichungstag der Anmeldung: 22.06.1988
(73) Patentinhaber: pvb medizintechnik gmbh, 85610 Kirchseeon (DE)
(72) Erfinder: Von Berg, Peter, D-8011 Neukeferloh (DE)
(74) Vertreter: von Bülow, Tam, Dr.

(56) Entgegenhaltungen:
- FR-A- 544 053
- FR-A- 2 366 504
- US-A- 4 335 729

## Beschreibung

Die Erfindung bezieht sich auf eine Dosiervorrichtung zur Einstellung der Durchflußrate einer innerhalb eines Ventilgehäuses in einem Strömungskanal in den oder aus dem Körper eines Lebewesens geführten Flüssigkeitsströmung, beispielsweise zur parenteralen oder intravenösen Infusion. Zur Änderung des Strömungswiderstands des Strömungskanals ist eine Stelleinrichtung vorgesehen, die durch ein Betätigungsorgan bedient werden kann. Eine solche Dosiervorrichtung ist aus der FR-A-2 366 504 bekannt und vermeidet die Nachteile einer bekannten Quetschrollen-Dosiereinrichtung.

Um die Stellmittel gegen unabsichtliches oder absichtliches Verstellen zu schützen, hat die Erfindung die Aufgabe, eine einen solchen Schutz bewirkende Sicherungseinrichtung fertigungstechnisch einfach zu realisieren, wobei die mühelose und genaue Einhandbedienung eines solchen gesicherten Ventils geschaffen werden soll.

Diese Aufgabe wird erfindungsgemäß durch den Gegenstand des Patentanspruchs 1 gelöst, also dadurch, daß die gattungsgemäße Dosiervorrichtung folgende weiteren Merkmale aufweist:
- eine Sicherungseinrichtung ist zum Schutz der Stelleinrichtung gegen unabsichtliche oder absichtliche Verstellung vorgesehen,
- die Sicherungseinrichtung ist im wesentlichen durch das Betätigungsorgan und die Stelleinrichtung gebildet und beide Teile sind hierzu sowie zu ihrem gegenseitigen lösbaren Eingriff nach Art eines Schlüssel-Schloß-Systems aneinander angepaßt, und
- das Betätigungsorgan ist aus einer konzentrisch zu einer Innenbüchse angeordneten kreiszylindrischen Außenbüchse aufgebaut und mit dem Ventilgehäuse ist ein Handgriff verbunden, der in einem solchen Abstand zur Außenbüchse angeordnet ist, daß dessen feinfühlige und mühelose Bedienung mit dem Daumen und Zeigefinger einer Hand erfolgen kann, während deren drei übrige Finger den Handgriff umgreifen, ohne daß gleichzeitig versehentlich der gegenseitige Eingriff von Betätigungsorgan und Stelleinrichtung gelöst wird.

Die erfindungsgemäße Sicherungseinrichtung hat gegenüber einer Sicherungseinrichtung, wie sie aus der DE 35 909 339 T1 bekannt ist, den Vorteil, daß außer den ohnehin herzustellenden Bauteilen, nämlich der Stelleinrichtung und deren Betätigungsorgan, keine weiteren Bauteile zu Sicherungszwecken hergestellt werden müssen. Außerdem ergibt sich gegenüber der bekannten Sicherungseinrichtung ein erhöhter Sicherungseffekt, da sich durch die wechselseitige Anpassung der Stelleinrichtung und der Betätigungsorgane nach Art eines Schloß-Schlüssel-Systems die Stelleinrichtung nur mit dem Betätigungsorgan bedienen läßt. Wird dieses vom Klinikpersonal wie ein Schlüssel aus einem Schloß abgezogen, kann die Einstellung der Stellmittel nicht mehr verändert werden.

Diese Sicherungseinrichtung ähnelt einem auf einem anderen technischen Gebiet aus der FR-A-544 053 bekannten Zapfhahn, bei dem das Betätigungsorgan und die von diesem drehbare Stelleinrichtung nach Art eines Schlüssel-Schloß-Systems aneinander angepaßt sind, um die unberechtigte Entnahme einer Flüssigkeit aus dem Ventil zu verhindern.

Damit für die das Dosierventil einstellende Person stets eine Hand für andere Arbeiten freibleibt, etwa für das Betätigen einer Blutdruck-Meßeinrichtung oder für das Halten eines Stethoskops, ist erfindungsgemäß der Handgriff so am Ventilgehäuse angebracht, daß der Daumen und der Zeigefinger an der Hand bequem das als geeignete Handhabe ausgebildete Betätigungsorgan erreichen können, wenn die drei übrigen Finger derselben Hand den Handgriff umgreifen.

Um zu vermeiden, daß dabei das lose aufgesetzte Betätigungsorgan ausser Eingriff mit dem Betätigungsanschluß an der Stelleinrichtung gelangt, ist es erfindungsgemäß durch eine geeignete Ausbildung von Betätigungsmechanismus und/oder Betätigungsorgan mit einem solchen Abstand vom Handgriff angeordnet, daß nicht nur die Bedienung mit Daumen und Zeigefinger der Bedienungsperson so mühelos erfolgen kann, daß das feinfühlige Einstellen des Dosierventils möglich ist, sondern gleichzeitig auch zuverlässig verhindert wird, daß während der Einstellung versehentlich das Betätigungsorgan vom zugehörigen Betätigungsanschluß gelöst wird.

Der erfindungsgemäß am Ventilgehäuse angebrachte oder angeformte Handgriff kann auch von einem Abstützelement eines gesonderten Stellantriebs etwa gabelartig umgriffen werden, so daß er gegebenenfalls für diesen als Drehmomentabstützung dienen kann.

Gemäß einer bevorzugten Ausgestaltung der Erfindung ist in dem vom Strömungskanal durchzogenen Ventilgehäuse ein darin bewegbar geführter Ventilkörper zur Veränderung des Strömungswiderstandes (Strömungskanalquerschnitt und/oder Strömungskanallänge) angeordnet. Das Betätigungsorgan ist hierbei mit demjenigen Ende des Körpers nach Art eines Schloß-Schlüssel-Systems für einen lösbaren Eingriff angepaßt, das vom Strömungskanal abgewandt ist. Hierdurch wird ein bequemer wechselseitiger Eingriff zwischen dem Betätigungsorgan und der Stelleinrichtung sichergestellt. Hierbei ist bevorzugt der Ventilkörper über ein Gewinde innerhalb des Gehäuses geführt und das Betätigungsorgan ist als Drehknopf ausgebildet, wie dies auch schon bei der eingangs genannten FR-A-2 366 504 der Fall ist.

Das Gewinde kann hierbei als Strömungskanal dienen. besonders dann, wenn es als Trapezgewinde ausgebildet ist.

Nach einer besonders einfachen Ausführungsform sind der Ventilkörper und das hierzu vorgesehene Betätigungsorgan für einen wechselseitigen Schnapp-Eingriff und/oder einen wechselseitigen Inbuseingriff (Innenmehrkant-Eingriff) aneinander angepaßt, wie dies für zwei miteinander lösbar zu verbindende Teile an sich bekannt und bewährt ist. Durch die Schnappverbindung kann der Ventilkörper innerhalb des Gehäuses nicht nur über eine Rotation um die Achse, sondern auch unmittelbar in axialer Richtung verschoben werden.

Bevorzugte Ausgestaltungen sind den Ansprüchen 2 bis 5 entnehmbar.

Zusätzlich ist es bevorzugt, daß der das Betätigungsorgan bildende Drehknopf auf seiner einen Seite eine zylindrische Ausnehmung aufweist, von deren Boden ein für den Eingriff mit dem Ventilkörper ausgebildeter erster Achsstummel koaxial vorsteht. Bevorzugt ist die Länge dieses ersten Achsstummels geringfügig kleiner-als die Tiefe der Ausnehmung, wobei weiter bevorzugt von der anderen Seite des Drehknopfes ein zweiter Achsstummel vorsteht, der für den gleichen Eingriff mit dem Ventilkörper wie der erste Achsstummel ausgebildet ist.

Bevorzugt sind Ventilgehäuse und Ventilkörper derart aneinander angepaßt, daß der Ventilkörper während sämtlicher zur Änderung des Strömungswiderstandes notwendiger Bewegungen stets innerhalb des Ventilgehäuses verbleibt.

Bevorzugt ist wenigstens der vom Strömungskanal abgewandte Endabschnitt des Ventilgehäuses aus einem elastisch verformbaren Material und zylindrisch ausgebildet, wobei der freie Rand des GehäuseEndabschnitts und der freie Rand der Drehknopf-Ausnehmung einen ringförmigen Wulst aufweist und die Durchmesser der Wulste so gewählt sind, daß der Gehäuse-Endabschnitt unter Schnappwirkung in die Drehknopf-Ausnehmung einführbar ist.

Die Innenwandung des Ventilgehäuses und/oder die Außenwandung des Ventilkörpers sind bevorzugt in jenem Bereich, in dem die beiden Anschlußstutzen des Ventilgehäuses zueinander versetzt sind, gewindeartig ausgebildet, und zwar auf eine solche Weise, daß der Gewindegang als Strömungskanal wirkt, dessen Länge durch eine axiale Verschiebung des Ventilkörpers im Ventilgehäuse änderbar ist.

Bei einem solchen Ventil kann der behandelnde Arzt das Betätigungsorgan mit sich führen, das nach Art eines Hauptschlüssels alle vorhandenen Ventile verstellen kann, während das Pflegepersonal nur solche Betätigungsorgane mit sich führt, die nur die ihm anvertrauten Dosierventile betätigen können.

Es könnte ein gesonderter und bevorzugt anatomisch ausgebildeter Handgriff am Ventilgehäuse angebracht werden, würde jedoch zu einer verhältnismäßig schweren Anordnung führen. Es ist daher bevorzugt stattdessen der Handgriff oder die Drehmomentabstützung vom verlängerten Zulauf- oder Ablaufstutzen gebildet. Dieser ist trotz seines in der Regel nur geringen Durchmessers als Handgriff völlig ausreichend, zumal meist zur Betätigung des Betätigungsorgans nur ein geringer Kraftaufwand erforderlich ist. Da das so weitergebildete Ventil sehr leicht ist, kann es auch frei in einer herabhängenden Infusionsleitung angebracht werden, ohne daß die Leitungsanschlüsse des Ventils an Zuverlässigkeit einbüßen.

Jedes Material, das eine einfache Herstellung, z.B. Kunststoff-Druckgießen, zuläßt und gegenüber der zu dosierenden Flüssigkeit hinlänglich inert ist, kann für das Ventilgehäuse verwendet werden. Bevorzugt wird jedoch ein durchsichtiges und weiter bevorzugt ein farbloses Material gewählt, so daß Anfang und Ende des Durchflusses und zum Teil auch die Durchflußmenge beobachtet werden können. Hierdurch wird visuell die genaue Einstellung des Ventils unterstützt, so daß die Einstellung selbst mit der gewöhnlich nicht bevorzugten, meist linken Hand hinlänglich feinfühlig erfolgen kann.

Bevorzugt ist der Ventilkörper oder der mit ihm bewegte Teil des Betätigungsmechanismus insgesamt oder mindestens im Bereich einer Markierung, insbesondere einer Skala, farblich so abgesetzt, daß er bzw. die Markierung von außen her ohne weiteres erkennbar ist. Bei der Einhandbedienung, bei welcher abwechselnd das Betätigungsorgan mit der linken oder rechten Hand betätigt wird, kann nämlich leicht die Drehrichtung verwechselt werden. Bei der genannten Ausgestaltung aber kann sofort und durch unmittelbare Beobachtung der Markierung bzw. des Ventilkörpers oder des Betätigungsmechanismus festgestellt werden, ob die Verstellung in der richtigen Richtung erfolgt oder nicht.

Es ist auch möglich, an der Außenseite des Ventilgehäuses und dem Ventilkörper, dem Betätigungsmechanismus oder einer Markierung gegenüberliegend eine optische Beobachtungseinrichtung vorzusehen, welches das Maß der Verstellbewegung ermitteln kann. Wenn nämlich das Ventil über eine flexible Antriebsverbindung mit einem gesonderten Stellantrieb verbunden ist, dann ist dessen Verstellweg, etwa die Anzahl der Umdrehungen, die der Stellantrieb zurücklegt, nicht immer repräsentativ für den Verstellweg des Ventilkörpers.

Es ist ferner möglich, jedem Durchsatz einer dosierten Flüssigkeit in Abhängigkeit von ihrer Viskosität einen Einstell-Erfahrungswert zuzuordnen, der bei der Handbetätigung des erfindungsgemäßen Dosierventils unmittelbar eingestellt oder als erster Sollwert in die Regelung eines Stellantriebs eingegeben wird. Auf diese Weise wird die für die Einstellung des Dosierventils erforderliche Zeitdauer noch weiter verkürzt.

Gemäß einer weiteren, bevorzugten Ausgestaltung der Erfindung ist im Dosierventil, und zwar im Ventilkörper, in der Ventilkammer oder im Ablaufstutzen eine Einrichtung zum Herbeiführen des Abtropfens der dosierten Flüssigkeit vorgesehen. Hierbei kann das Ansprechverhalten des Dosierventils auf die jeweils vorgenommene Einstellung besonders rasch überwacht werden, so daß eine noch genauere und raschere Einstellung eines gewünschten Durchsatzes ermöglicht wird.

Dabei ist die Länge des Ablaufstutzens, der bevorzugt den Handgriff bildet, so weit vergrößert, daß die Abtropfstelle nicht von der das Dosierventil haltenden Hand bedeckt wird.

Schließlich ermöglicht die Durchsichtigkeit des Dosierventils auch den Einsatz einer optisch wirkenden Beobachtungseinrichtung zur Durchflußmengenregistrierung, insbesondere Tropfregistrierung. Die Beobachtungseinrichtung ist bevorzugt ausgangsseitig an eine Überwachungseinrichtung oder an die Steuerung des Stellantriebs angeschlossen und spricht dann an, wenn der Ist-Wert der Durchflußmenge oder der Tropfzahl infolge irgendeiner Störung, etwa durch Zusetzen der Ventilkammer trotz geöffneten Dosierventils, vom Sollwert um einen vorgegebenen Wert abweicht. Die Überwachungseinrichtung kann hierbei als Lichtschranke oder als Tropfenzähler ausgebildet sein.

Der Betätigungsanschluß des eingangs genannten Ventils ist als Innensechskant ausgebildet. Der Betätigungsanschluß kann aber allgemein als unrunder Zapfen ausgebildet sein, und zwar bevorzugt als Vierkant- oder Sechskantzapfen. Der besondere Vorteil dieser Ausgestaltung liegt darin, daß zum Anschluß eines gesonderten Stellantriebes als biegsame Welle zum Ausgleichen von Fluchtfehlern zwischen der Ausgangswelle des Stellantriebs und dem Betätigungsanschluß einfach ein Schlauch verwendet werden kann, der über den unrunden Zapfen geschoben wird. Das Betätigungsorgan ist mit einer analogen, unrunden Bohrung ausgebildet, die bevorzugt als Durchgangsbohrung ausgebildet ist. Diese hat nicht nur den Zweck, einen Lufteinschluß beim Aufschieben des Betätigungsorgans auf den unrunden Zapfen zu vermeiden, sondern ermöglicht auch den Anschluß eines Stellantriebes, dessen Ausgangswelle als ein ebenso wie der Betätigungsanschluß ausgebildeter unrunder Zapfen geformt ist.

Hierbei ist eine nahezu torsionsfreie Verbindung zwischen dem Betätigungsanschluß und dem Stellantrieb hergestellt, so daß zur Regelung der Einstellbewegung des Ventilkörpers eine Meßgröße aus dem Stellantrieb verwendet werden kann, etwa die Anzahl der von diesem zurückgelegten Umdrehungen. Hierbei ist bevorzugt im Stellantrieb ein selbstsperrendes Getriebe vorgesehen, das die Drehung der Ausgangswelle des Stellantriebs und somit auch des Betätigungsorganes mit dessen Handhabe nur dann zuläßt, wenn diese Drehung durch den Stellantrieb selbst herbeigeführt wird. Eine unbefugte Verstellung des Stellantriebs von Hand ist in diesem Fall nicht möglich.

Bei der Verwendung eines Schlauchs als biegsame Welle wird das Betätigungsorgan weggelassen. In diesem Fall ist ebenfalls die Verstellung des Ventils durch Unbefugte praktisch ausgeschlossen, weil kein Betätigungsorgan vorliegt, dessen Handhabe erkennbar zum Verstellen des Dosierventils geeignet wäre.

Es ist grundsätzlich möglich, bei Verwendung einer Gewindeanordnung als Betätigungsmechanismus den Anschluß des Ventilkörpers an der Ventilkammer als Endanschlag für die Schließstellung des Ventiles zu verwenden. Da aber bevorzugt der Schließkörper und die Ventilkammer konisch ausgebildet sind, würden in diesem Fall unter Umständen sehr hohe Klemmkräfte auftreten, welche unter Umständen die Sprengung der Ventilkammer herbeiführen könnten. Aus diesem Grund ist gemäß einer weiteren, bevorzugten Ausgstaltung der Erfindung die Gewindeanordnung so ausgebildet, daß sie bei vollem Einschrauben des Ventilkörpers einen Endanschlag bildet, wenn sich der Ventilkörper in seiner Schließstellung befindet. Hierbei kann durchaus noch ein Kapillarspalt um den Ventilkörper herum bestehen, ohne daß es zu einer Strömung kommt. Das erfindungsgemäße Dosierventil ist nämlich regelmäßig nur einem geringen Druckgefälle von allenfalls 1 m WS (Wassersäule) ausgesetzt, so daß wegen der Viskosität der regelmäßig verwendeten Flüssigkeiten eine Strömung durch einen Kapillarspalt niht zustande kommt.

Bei der Montage wird ein solcher Ventilkörper von außen her mittels eines Außengewindes in das Innengewinde im Ventilgehäuse eingeschraubt und kann mithin in umgekehrter Richtung auch wieder aus diesem herausgeschraubt werden. Gemäß einer weiteren, bevorzugten Ausgstaltung der Erfindung ist auch in der Öffnungslage des Ventilkörpers ein Endanschlag vorgesehen. Dieser verhindert, daß der Ventilkörper versehentlich herausgeschraubt werden kann. Besonders bei Einhandbedienung ist es nämlich möglich, daß bei der Drehbetätigung des Betätigungsorganes dieses zunächst in der falschen Richtung verdreht wird, bis der Gewindeeingriff aufgehoben ist, Es genügt dann nur noch eine ungeschickte Bewegung, um den Ventilkörper aus dem Ventilgehäuse fallenzulassen. Die Folge wäre das Ablaufen der zu dosierenden Flüssigkeit aus der Öffnung, in welcher der Betätigungsanschluß normalerweise die Wand des Ventilgehäuses durchdringt. Ferner kann durch diese Öffnung auch Luft in den Ablaufstutzen gelangen. Diese Störungen werden durch die erfindungsgemäße Weiterbildung vermieden.

Der Endanschlag in der Öffnungsstellung des Ventilkörpers ist auch bei Verwendung eines Stellantriebes wichtig: sollte durch ein Versagen der Regelung, das grundsätzlich einen Alarm auslöst, dennoch der Stellantrieb zunächst ohne Unterbrechung weiterlaufen, dann wird durch diese Ausgestaltung verhindert, daß sich der Ventilkörper aus dem Ventilgehäuse lösen kann, bevor eine Kontrollperson eingegriffen hat.

Bei dem eingangs genannten, bekannten Dosierventil ist das Betätigungsorgan gleitend am Ventilgehäuse geführt. Hierbei ist es dann, wenn die eine Hand das Gehäuse und die andere das Betätigungsorgan ergreift, ohne weiteres möglich, trotz des Übergangs von ruhender zu gleitender Reibung auch sehr kleine Einstell-Drehbewegungen durchzuführen. Dies ist bei der Einhandbetätigung nicht so ohne weiteres möglich. Deshalb ist gemäß einer weiteren, bevorzugten Ausgestaltun der Erfindung am Ventilgehäuse dem als Drehelement bzw. Drehknopf ausgebildeten Betätigungsorgan gegenüberliegend ein Lagerkörper angeordnet, von dem dieser Drehknopf geführt ist und mit dem dieser Drehknopf mithin in Reibungseingriff steht. Dieser Lagerkörper ist aus einem Material gebildet, das in Paarung zu dem Material des Drehknopfes einen so geringen Reibungsbeiwert aufweist, daß der beim Übergang von der ruhenden zur gleitenden Reibung auftretende Ruck nur außerordentlich gering ist. Hierdurch wird die Verstellung des Drehknopfes um einen nur sehr kleinen Drehwinkel auch bei er Einhandbedienung ohne weiteres möglich.

Das Material des Lagerkörpers ist bevorzugt selbstschmierender bzw. ein solcher Kunststoff, der einen nur sehr geringen Reibungsbeiwert gegenüber anderen Stoffen liefert. Solche Stoffe sind dem Fachmann bekannt, so daß er sie den Erfordernissen entsprechend ohne weiteres auswählen kann.

Der Lagerkörper kann auch als eine auf das Ventilgehäuse und/oder auf das Betätigungsorgan im Bereich des Reibungseingriffes aufgebrachte Beschichtung ausgebildet sein.

Der Lagerkörper ist bevorzugt als Lagerbüchse ausgebildet, die von außen her in die vom Betätigungsanschlub durchdrungene Öffnung des Ventilgehäuses eingesetzt ist. Dabei weist die Lagerbüchse einen einwärts gerichteten Flansch auf, der vom Betätigungsanschluß drehbar durchsetzt ist.

Wenn gemäß der oben erwähnten, bevorzugten Ausgestaltung der Betätigungsanschluß als unrunder Zapfen ausgebildet ist, dann weist das Betätigungsorgan eine entsprechende Aufnahmebüchse auf, die über den Zapfen aufsteckbar ist und dann an ihrem Außenumfang in der Lagerbüchse geführt ist. Diese Anordnung ermöglicht nicht nur die mühelose Verstellbewegung des Betätigungsorgans, sondern erleichtert gleichzeitig auch das Aufsetzen des Betätigungsorganes auf den Betätigungsanschluß. Diese beiden Elemente sind nämlich durch die Lagerbüchse zentriert. Hierbei sind diese Elemente so aufeinander abgestimmt und bemessen, daß bei ihrem gegenseitigen Auf- oder Ineinanderschieben ein Eingriff zwischen der Lagerbüchse und dem Betätigungsorgan eintritt, bevor letzteres mit dem Betätigungsanschluß in Eingriff tritt.

Die Lagerbüchse kann zur Außenseite des Ventilgehäuses überstehen. Das Betätigungsorgan trägt einen gerippten oder geriffelten, also mit Handhaben versehenen, Dichtring, der über einen Flansch mit einer auf den Betätigungsanschluß aufsetzbaren, zum Drehring konzentrischen Innenbüchse verbunden ist. Wenn dieser Flansch nun gegen die Stirnseite der Lagerbüchse anliegt, dann wird auch die Reibung zwischen diesen Elementen verringert, so daß insgesamt das feinfühlige Verdrehen des Betätigungsanschlusses noch weiter erleichtert wird.

Gemäß einer weiteren, bevorzugten Ausgestaltung der Erfindung ist der vom Betätigungsanschluß durchsetzte Durchbruch dieser Lagerbüchse so bemessen, daß er kleiner ist als das an der Verlängerung des Ventilkörpers ausgebildete Außengewinde. Gemäß der bevorzugten Ausgestaltung ist die Lagerbüchse ihrerseits im Ventilgehäuse befestigt, und zwar vorzugsweise eingeklebt, so daß sie den Endanschlag für den Ventilkörper in dessen Öffnungsstellung bietet und das völlige Herausschrauben des Ventilkörpers ohne weiteres verhindert.

An einem Material mit guten Gleiteigenschaften, wie es das Material der Lagerbüchse ist, haften viele Kleber nur schlecht. Zur Befestigung der Lagerbüchse im Ventilgehäuse ist deshalb bevorzugt ein Kleber gewählt, der die Materialien dieser beiden Körper oberflächlich auflöst, so daß er eine zuverlässige und feste Verbindung zwischen diesen herstellt.

Es ist auch möglich, einen Kleber zu wählen, der nur mit einem dieser beiden Körper eine feste Verbindung eingeht, aber seinerseits voll aushärten kann. Beim anderen der genannten Teile sind dann hinterschnittene Aussparungen gebildet, die von dem Kleber beim Zusammenbau ausgefüllt werden. Der ausgehärtete Kleber verhindert somit ebenfalls das Lösen der genannten Teile. Solche Aussparungen können auch in beiden Teilen gegenüberliegend vorgesehen sein, wobei als Kleber lediglich ein Material gewählt ist, das ausreichend aushärtet. Auch in diesem Fall sind die beiden Teile unlösbar miteinander verbunden.

Die Gefahr einer groben Fehlbedienung durch ungeübtes oder ungeschicktes Personal kann beim erfindungsgemäßen Dosierventil ebenso wenig völlig ausgeschlossen werden, wie bei einem herkömmlichen Dosierventil. Ebenso wenig kann mit letzter Sicherheit ohne großen technischen Aufwand ausgeschlossen werden, daß ein etwa vorhandener Stell- oder Regelantrieb zum Einstellen des Dosierventils nicht doch einmal so versagt, daß er das Dosierventil in unzulässiger Weise weit öffnet. Schließlich kann auch nicht völlig ausgeschlossen werden, daß die über das Dosierventil verabreichte Flüssigkeit unerwartet plötzlich unterbrochen werden muß, der behandelnde Arzt aber das Betätigungsorgan für den Ventilkörper nicht zur Hand hat.

Derartige Situationen bilden besonders bei der Verabreichung solcher Medikamente eine Gefahr, bei denen eine Überdosierung sorgsam vermieden werden sollte.

Aus diesem Grund ist, um das Dosierventil für die Einhandbedienung ohne die Einstellung mittels eines Fremdantriebes noch geeigneter zu machen, gemäß einer weiteren Ausgestaltung der Erfindung vorgeschlagen, das erfindungsgemäße Dosierventil mit einer zusätzlichen Absperreinrichtung insbesondere für den Notfall auszustatten.

Diese Absperreinrichtung ist bevorzugt dem Ventilkörper nachgeschaltet, um etwa bei Zerstörung des Ventilkörpers durch grob unsachgemäße Bedienung das Eindringen von Krankheitserregern, Luft und dergleichen in die zum Patienten führende Schlauchleitung zu verhindern.

Die Absperreinrichtung kann vorteilhafterweise ein kraftgesteuertes, von außen her ansteuerbares Absperrventil sein, das etwa durch einen Elektromagneten ansteuerbar ist. Der Vorteil dieser Ausgestaltung liegt darin, daß die Absperreinrichtung im Notfall auch von der Regelung des Fremdantriebes her angesteuert werden kann, wenn etwa diese Regelung feststellt, daß der von ihr ständig überwachte Fremdantrieb aus der Steuerung läuft.

Gemäß einer bevorzugten Ausgestaltung der Erfindung ist die Absperreinrichtung jedoch als baulich sehr einfache Schlauchklemme ausgebildet, mit zwei Klemmschenkeln, die gabelartig angeordnet sind und zwischeneinander einen Klemmschlitz begrenzen, dessen Breite gerade so bemessen ist, daß der jeweils verwendete Schlauch in diesen Schlitz eingeschoben und hierbei wirksam abgeklemmt werden kann.

Soweit nämlich des erfindungsgemäße Dosierventil nur für die Bedienung von Hand ausgelegt ist, ist es von besonderem Vorteil, die Absperreinrichtung so leicht wie nur irgend möglich auszubilden, um nicht durch ein zu hohes Gewicht dieser Absperreinrichtung den Sitz der Schlauchanschlüsse an dem Dosierventil zu beeinträchtigen.

Die Anordnung der Schlauchklemme am Handgriff hat noch weiter den Vorteil, daß der Schlauch gegebenenfalls von den den Handgriff haltenden Fingern, falls erforderlich, in den Klemmschlitz gedrückt werden kann.

Um dies noch zu erleichtern, ist gemäß einer weiteren, bevorzugten Ausgestaltung der Erfindung die Schlauchklemme an der vom Betätigungsorgan abgewandten Seite des Handgriffs angebracht bzw. angeformt ist, wobei der Handgriff, wie bereits schon erörtert, als Auslaßstutzen ausgebildet werden kann. Ferner sind bevorzugt die Klemmschenkel an ihren freien Enden zum Klemmschlitz hin abgeschrägt, wobei sie nicht nur eine Führung beim Einlegen des Schlauchs in den Klemmschlitz bilden, sondern auch einen Schlauchsitz: bei der Bedienung des erfindungsgemäßen Dosierventils wird hierbei der Handgriff von der Hand erfaßt, wobei der Schlauch in dem am Ende der Klemmschenkel gebildeten Sitz angeordnet ist. Wenn nun der Benutzer die Flüssigkeitsströmung plötzlich unterbrechen will, genügt ein kurzes Andrücken des Schlauches mit der ihn gemeinsam mit dem Handgriff haltenden Hand in Richtung gegen den Klemmschlitz, um den Schlauch sicher in den Klemmschlitz zu führen und die sofortige Absperrung der Medikamentenzufuhr zum Patienten zu erreichen. Durch Einführen eines Fingers zwischen Schlauch und Gehäuse kann der Schlauch mühelos mit einer Hand wieder aus dem Klemmschlitz gehoben werden. So lange sich der Schlauch im Klemmschlitz befindet, kann die Einstellung des Betätigungsorgans vorgenommen werden, ohne daß deshalb dem Patienten zu viel Medikament zugeführt wird.

Ein weiterer Vorteil der erfindungsgemäßen Schlauchklemme liegt darin, daß kurzzeitig die Medikamentenzufuhr zum Patienten völlig unterbrochen werden kann, etwa dann, wenn es erforderlich ist, den Sitz der das Medikament in den Körper des Patienten einführenden Kanüle zu korrigieren, ohne daß es gleichzeitig erforderlich ist, die getroffene genaue und zutreffende Einstellung des Betätigungsorgans und somit des Ventilkörpers ändern.

Der Gegenstand der Erfindung wird anhand der beigefügten, schematischen Zeichnung beispielsweise noch näher erläutert.

In dieser zeigt:
- Fig. 1: eine Schnittdarstellung einer ersten Ausführungsform der erfindungsgemäßen Dosiervorrichtung,
- Fig. 1a: eine teilweise geschnittene Ansicht einer abgewandelten Stelleinrichtung nach Fig. 1 mit einem gewindeartigen Abschnitt des Strömungskanals am Ventilkörper,
- Fig. 2a: eine teilweise geschnittene Ansicht einer gegenüber fig. 1a abgewandelten Stelleinrichtung mit einem zylindrischen Endbereich des Ventilkörpers,
- Fig. 2b: eine teilweise geschnittene Ansicht einer gegenüber Fig. 2a abgewandelten Stelleinrichtung mit einer gewindeartigen Verlängerung des Strömungskanals im zylindrischen Endbereich des Ventilkörpers,
- Fig. 2c: eine teilweise geschnittene Ansicht einer gegenüber den Fig. 2a und 2b abgewandelten Stelleinrichtung mit einem gewindeartigen Strömungskanal sowohl im zylindrischen Endbereich als auch im mittleren Bereich des Ventilkörpers,
- Fig. 3: einen dreieckigen Innenquerschnitt des Anschlußstutzens,
- Fig. 4: ein erfindungsgemäßes Dosierventil im Querschnitt und in Explosionsdarstellung,
- Fig. 5: das Dosierventil der Fig. 4 im Querschnitt und in montiertem Zustand,
- Fig. 6: die Ansicht des Gehäuse gemäß einer anderen Ausführungsform der Erfindung, mit einer angeformten Schlauchklemme,
- Fig. 7: den Schnitt IV-IV in Fig. 6, und
- Fig. 8: die Ansicht V in Fig. 6.

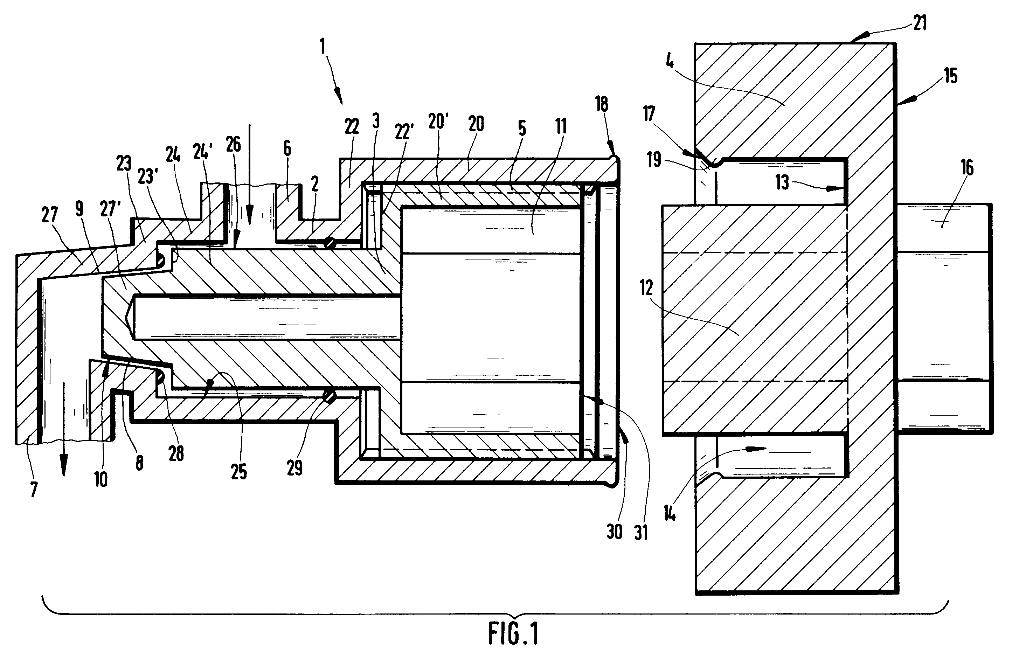

Die in Fig. 1 dargestellte erste Ausführungsform der Dosiervorrichtung bzw. des Dosierventils besteht aus einer Stelleinrichtung 1 und einem Betätigungsorgan 4 für die Stelleinrichtung. Die Stelleinrichtung 1 besteht aus einem Gehäuse 2 und einem darin beweglich gelagerten und geführten Körper 3. Das Gehäuse 2 ist nach Art eines Ventilgehäuses und der Körper 3 nach Art eines Ventilkörpers aufgebaut. Das Gehäuse 2 und der Körper 3 sind im wesentlichen nach Art von stufenförmig sich verjüngenden Hohlzylindern aufgebaut und koaxial ineinander angeordnet.

Die drei den Zylinderstufen entsprechenden Abschnitte 20, 24 und 27 des Gehäuses 2 gehen über die Absätze 22 und 23 ineinander über. Entsprechend gehen die drei Abschnitte 20ʹ, 24ʹ, 27ʹ des Körpers 3 über die beiden Absätze 22ʹ und 23ʹ ineinander über.

Des Körper 3 ist über ein Gewinde 5 im Gehäuse 2 drehbar gelagert und geführt. Das Gewinde 5 ist im ersten Abschnitt 20 bzw. 20ʹ des Gehäuse 2 bzw. des Körpers 3 angeordnet.

Der sich an den ersten Abschnitt 20 des Gehäuse 2 anschließende zweite Abschnitt 24 ist mit einem Anschlußstutzen 6 bestückt. Der sich an den zweiten Abschnitt 24 anschließende dritte Abschnitt 27 des Gehäuses 2 ist mit einem weiteren Anschlußstutzen 7 bestückt. Die beiden Anschlußstutzen 6 und 7 sind demnach in Gehäuseachsrichtung gegeneinander versetzt angeordnet und dienen als Schlauchanschlüsse für die Zu- und Ableitung von Flüssigkeiten in die und aus der Dosiervorrichtung, beispielsweise Plasma, Antibiotika etc. Beim dargestellten Ausführungsbeispiel strömt die Flüssigkeit durch den Anschlußstutzen 6 in den Zwischenraum zwischen Gehäuse 2 und Körper 3 ein und aus dem Anschlußstutzen 7 wieder aus. Der Querschnitt dieses Verbindungskanals läßt sich in einem Kanalabschnitt, im folgenden Strömungskanal 8 genannt, ändern. Dieser Strömungskanal 8 wird dabei von einer der Innenwandung des Gehäuseabschnitts 27 ausgebildeten Konusfläche 9 und einer in der Außenwandung des dritten Körperabschnitts 27ʹ entsprechend ausgebildeten Konusfläche 10 begrenzt.

In dem vom Strömungskanal 8 abgewandten Endabschnitt 20ʹ des Körpers 3 ist ein sich in Achsrichtung erstreckender Inneninbus 11 ausgeformt, der mit einem hierzu komplementären Außenimbus 12 am Drehknopf 4 in Eingriff bringbar ist. Bei gegenseitigem Eingriff von Innen- und Außeninbus 11 und 12 kann durch Drehen des Drehknopfs 4 der Querschnitt des Strömungskanals 8 verändert werden.

Der Außeninbus 12 ist nach Art eines ersten Achsstummels zentrisch am Boden 13 einer zylindrischen Ausnehmung 14 im Drehknopf 4 angeordnet. Am freien Rand der Ausnehmung 14 ist eine Fase 17 eingearbeitet, deren mittlerer Durchmesser größer ist als der Durchmesser eines längs des freien Randes des Gehäuses 2 ausgebildeten ersten ringförmigen Wulstes 18. An die Fase 17 der Ausnehmung 14 schließt sich ein zweiter ringförmiger Wulst 19 an. Im ineinandergesteckten Zustand von Stelleinrichtung 1 und Drehknopf 4 bilden die beiden Wulse 18 und 19 einen Spezialanschlag.

Von der der zylindrischen Ausnehmung 14 abgewandten Bodenseite 15 des Drehknopfs 4 steht ein Außeninbus 16 nach Art eines zweiten Achsstummels zentrisch vor und ist ebenfalls für einen Eingriff mit dem Inneninbus 11 ausgeformt. Schließlich ist in der zylindrischen Mantelfläche des Drehknopfs 4 eine Drehknopfrändelung 21 zu dessen leichterer Drehbetätigung von Hand vorgesehen.

Im ringförmigen Absatz 23 des Gehäuses 2 ist ein Dichtring 28 zur vollständigen Schließung des Verbindungskanals zwischen den Anschlußstutzen 6 und 7 angeordnet. Ein zwischen dem Absatz 22 des Gehäuses 2 und dem Anschlußstutzen 6 angeordneter O-Ring 29 verhindert das Entweichen der Flüssigkeit in Richtung des ersten Gehäuseabschnitts 20.

Im Betriebszustand strömt die in den Körper eines Lebewesens einzuführende Flüssigkeit vom Anschlußstutzen 6 zum Anschlußstutzen 7. Durch Axialverschiebung des Körpers 3 innerhalb des Gehäuses 2 kann der Querschnitt des konischen, ringförmigen Strömungskanals 8 geändert werden. Hierzu werden der Inneninbus 11 des Stützkörpers 3 un einer der beiden Außeninbusse 12 oder 16 des Drehknopfs 7 in Wirkeingriff miteinander gebracht und dann der Körper 3 im Gehäuse 2 über das Gewinde 5 verdreht. Diese Drehung bewirkt eine Axialverschiebung des Körpers 3 im Gehäuse 2, die ihrerseits zu einer Querschnittsveränderung des Strömungskanals 8 und damit zu einer Änderung der Durchflußrate führt.

Soll der in der Ausnehmung 14 des Drehknopfs 4 angeordnete Inbus 12 zur Änderung des Strömungsquerschnitts verwendet werden, muß der Drehknopf 4 auf den ersten Abschnitt 20 des Gehäuses 2 aufgeschoben werden. Das Aufschieben des Drehknopfs 4 auf den zylindrischen Gehäuseabschnitt 20 mit seiner am äußeren Randd befindlichen Wulst 18 wird durch die Fase 17 am äußeren Rand der Drehknopfaufnehmung 14 und durch Verwendung eines elastisch verformbaren Materials erleichtert. Das unabsichtliche Herunterrutschen des Drehknopfs 4 vom zylindrischen Gehäuseabschnitt 20 und von Inbus 11 wird durch die Wülste 18 und 19 verhindert.

Zur Absicherung gegen absichtliches oder unabsichtliches Verstellen dr Dosiervorrichtung wird der Drehknopf 4 von der aus Gehäuse 2 und Körper 3 gebildeten Stelleinrichtung 1 abgezogen. Wegen der elastischen Verformbarkeit des Drehknopfes 4 und/oder der Stelleinrichtung 1 ist dies trots des aus Wulst 18 und 19 gebildeten Axialanschlages möglich. Da die Stirnfläche 31 des Körpers 3 nicht nach außen über die Stirnfläche 30 des Gehäuses 2 hinausragt, ist es einem unbefugten Bediener der Stelleinrichtung 1 ohne den Drehknopf 4 nicht möglich, den Körper 3 zu drehen und damit den Strömungskanal 8 zu verändern. Will dagegen der befugte Bediener der Dosiervorrichtung kurzfristig eine Veränderung der Durchflußrate vornehmen, so verwendet er zur Betätigung des Körpers 3 den kurzen Außenimbus 16 an dem von der Ausnehmung abgewandten Drehknopfboden 15. Dadurch ist ein besonders schnelles Einführen des Außenimbus 16 in den Innenimbus 11 nach Art eines Schlüssel-Schluß-Systems und damit eine besonders schnelle Betätigung der Dosiervorrichtung möglich.

Bei der Ausführungsform nach Fig. 1a liegt die Körpermantelfläche 26 des zweiten, d.h. mittleren zylindrischen Körperabschnitts 24ʹ an der Gehäuseinnenwandung 25 des mittleren Gehäuseabschnitts 24 drehbar gelagert an. In diesem Abschnit ist die Mantelfläche 26 des Körpers 3 von einem gewindeartigen Strömungskanal 32 durchzogen. Die den Strömungskanal 32 bildenden Gewindegänge haben konstanten Querschnitt. In diesem Ausführungsbeispiel strömt die Flüssigkeit über den Anschlußstutzen 6 durch den gewindeartigen Strömungskanal 32 bis zum ringförmigen, kegelförmig sich verjüngenden Strömungskanal 8, um dann über den Anschlußstutzen 7 in den Körper des Lebewesens fließen zu können.

Durch Drehung des in dieser Abbildung nicht mehr dargestellten Drehknopfs 4 wird der Körper 3 durch den Inbus-Gegeninbus-Eingriff in axialer Richtung verschoben. Hierdurch ändert sich nicht nur der Querschnitt des Strömungskanals 8 (wurde bereits anhand der Fig. 1 beschrieben), sondern auch die Länge des gewindeartig angeordneten rillenförmigen Strömungskanals 32. Je nach Lage des Körpers 3 gegenüber dem Gehäuse 2 liegen nämlich zwischen dem Anschlußstutzen 6 und dem Strömungskanal 8 unterschiedlich viele Gewindegänge. Durch Änderung der Länge des gewindeartigen Strömungskanal 32 wird dessen Strömungswiderstand geändert (wachsender Strömungswiderstand mit wachsender Strömungskanallänge).

Mit Ausnahme der mittleren Abschnitte 24 bzw. 24ʹ des Gehäuses 2 bzw. des Körpers 3 gleicht das in Fig. 1a dargestellte Ausführungsbeispiel dem in Fig. 1 dargestellten Ausführungsbeispiel.

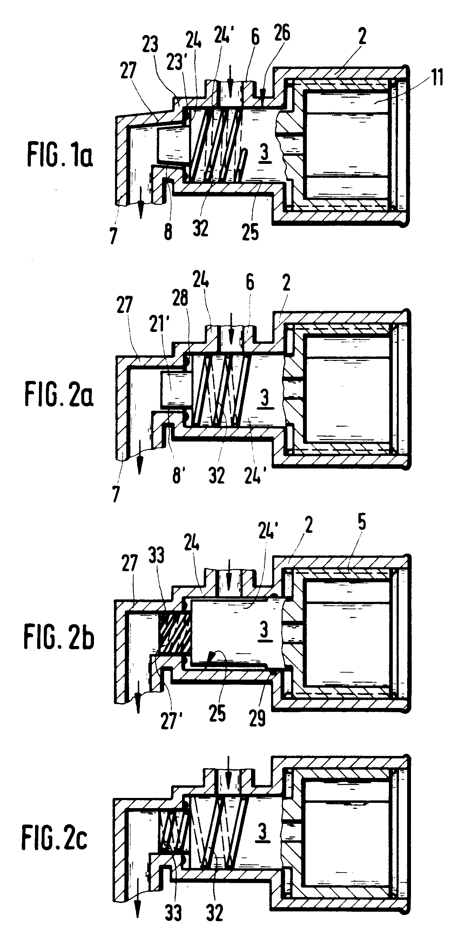

Das in Fig. 2a dargestellte Ausführungsbeispiel unterscheidet sich im wesentlichen durch folgende Merkmale von dem in Fig. 1a dargestellten Ausführungsbeispiel:
Die dritten Abschnitte 27 bzw.27ʹ des Gehäuses 2 bzw. des Körpers 3 sind nicht konisch, sondern zylindrisch ausgebildet.

Demgemäß verändert sich der Querschnitt des Strömungskanals 8ʹ nicht bei einer axialen Verschiebung des Körpers 3. Bei diesem Ausführungsbeispiel hat demnach der Strömungskanal 8ʹ stets konstanten Querschnitt. Der Strömungswiderstand des gesamten Strömungskanals d.h. der Strömungskanäle 8ʹ und 32ʹ, wird demnach lediglich durch Änderung von dessen Länge geändert.

Durch zylindrische Ausbildung der beiden dritten Abschnitte 27, 27ʹ sind Gehäuse 2 und Körper 3 fertigungstechnisch leichter herstellbar. Von Vorteil ist auch, daß grundsätzlich keiner O-Ring-Dichtung zwischen der Außenwandung des mittleren Körperabschnitts 24ʹ und des mittleren Gehäuseabschnitts 24 bedarf.

Schließlich ist als Vorteil noch die im Vergleich zu Fig. 1 präzisere Lagerung des Körpers 3 im Gehäuse 2 anzumerken. Der Körper 3 ist nämlich nicht nur über das Gewinde 5, sondern auch über seinen Mittelabschnitt 24ʹ im Gehäuse 2 geführt. Diese Vorteile gelten im übrigen auch für das Ausführungsbeispiel nach Fig. 1a.

In Fig. 2b ist eine ähnliche Ausführungsform wie in Fig. 2a dargestellt. Hier befindet sich der gewindeartige Strömungskanal 33 nicht im mittleren zylindrischen Abschnitt 24 des Körpers 3, wie etwa in Fig. 2a, sondern im dritten zylindrischen Abschnitt 27. Der mittlere, d.h. zweite zylindrische Abschnitt 24ʹ des Körpers 3 hat einen geringeren Durchmesser als der Durchmesser der Gehäuseinnenwandung 25. Wie in Fig. 1 ist auch hier gegen ein Entweichen der Flüssigkeit in Richtung des Gewindes 5 ein O-Ring vorgesehen.

Ein Vorteil dieser Ausführungsform ist die besonders präzise Lagerung und Führung des Körpers 3 im Gehäuse 2. Dadurch kann auch bei Erschütterung der Dosiervorrichtung ein unabsichtliches Verstellen des Körpers 3 gegenüber dem Gehäuse 2 weitgehend vermieden werden. Auch bei diesem Ausführungsbeispiel wird der Strömungswiderstand lediglich durch Änderung des Strömungskanals 30 geändert.

In Fig. 2c sind die Prinzipien in den Fig. 2a und 2b gemeinsam verwirklicht. Ein gewindeartiger Strömungskanal 32, 33 befindet sich sowohl im zweiten als auch im dritten zylindrischen Abschnitt 24ʹ und 27ʹ des Körpers 3.

Ist - entgegen Fig. 1 - die Länge des Inbus 12 kürzer als die Tiefe der ihn umgebenden Ausnehmung 14, dann ist dessen freie Stirnfläche vor Beschädigungen weitgehend gesichert.

Die gewindeartigen Strömungskanäle können grundsätzlich auch in der Innenwandung des zweiten und/oder dritten Abschnitts 24 und 27 des Gehäuses 2 ausgeformt sein, und nicht in den entsprechenden Außenwandabschnitten des Körpers 3. Stattdessen können sie auch zu einem für das Gehäuse 2 und den Körper 3 gemeinsamen Gewinde, insbesondere Trapezgewinde, ausgebildet sein. Diese Ausführungsform hat den Vorteil, daß der gewindeartige Strömungskanal gleichzeitig als Schraubgewinde zur Führung und Verstellung des Körpers 3 im Gehäuse 2 dient und daher grundsätzlich auf das Gewinde 5 verzichtet werden kann.

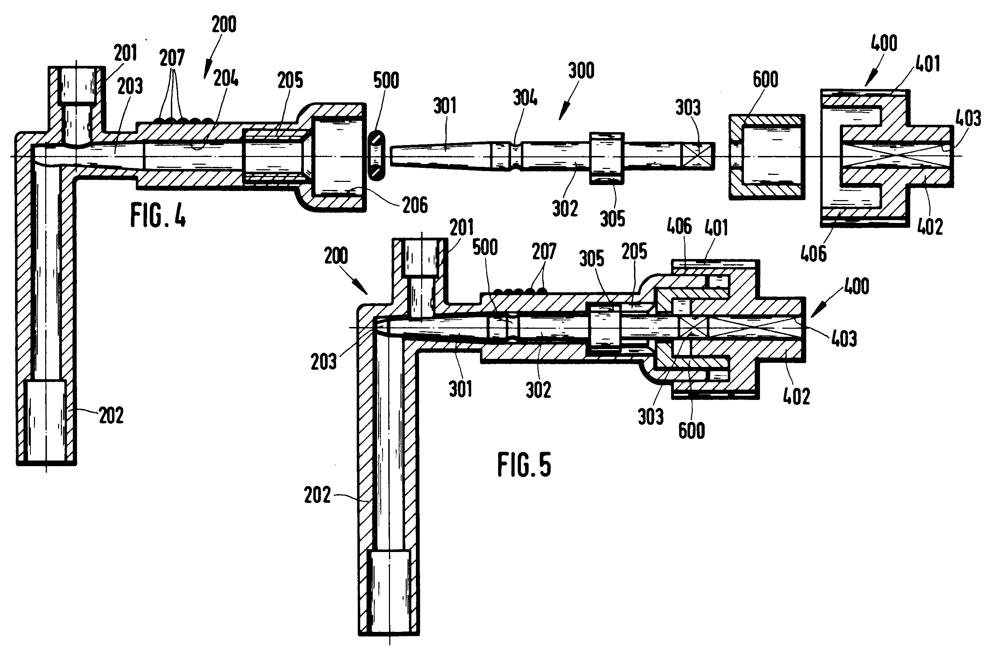

In Fig. 3 ist eine besonders vorteilhafte Ausführungsform des Anschlußstutzens 6 im Querschnitt dargestellt. Der Innenquerschnitt 34 des Anschlußstutzens 6 verjüngt sich in Richtung zum dritten Abschnitt 27 des Gehäuses 2. Vorzugsweise, wie hier dargestellt, hat der Innenquerschnitt die Form eines Dreiecks, besonders bevorzugt eines gleichschenkligen Dreiecks. Diese dreieckige Innenquerschnittsform 34 des Anschlußstutzens 6 setzt sich durch die Wandung des zweiten Abschnittes 24 hindurch biz zum Einlaß in den Zwischenraum zwischen Gehäuse 2 und Körper 3 fort.

Durch diese Art der Ausbildung des Innenquerschnitts 34 des Anschlußstutzens 6 wird je nach Stellung des Körpers 3 mit einem Absatz 23ʹ gegenüber der Dreiecksfläche im Gehäuse 2 eine weitere Reguliermöglichkeit des Durchflusses der Dosiervorichtung eröffnet, insbesondere die Möglichkeit einer Feinregulierung im Bereich feinerer Durchflußraten und einer Schnellregulierung im Bereich großer Durchflußraten.

Grundsätzlich genügt es, den oben beschriebenen Innenquerschnitt 34 nur im Mündungsbereich vorzusehen, d.h. in demjenigen Endabschnitt des Kanals 6, der den Körper 3 unmittelbar gegenüberliegt.

Das Dosierventil der Fig. 4 bis 8 weist drei Hauptteile auf, und zwar
- ein Ventilgehäuse 200
- einen Ventilkörper 300 und
- ein Betätigungsorgan 400.

Ferner sind ein Dichtungsring 500 und eine Lagerbüchse 600 vorgesehen.

Das Ventilgehäuse 200 weist einen Einlaßstutzen 201 und einen Auslaßstutzen 202 auf. Diese beiden Stutzen 201, 202 sind achsparallel zueinander angeordnet, jedoch zueinander versetzt. Der Auslaßstutzen 202 weist eine erheblich größere Länge als der Einlaßstutzen 201 auf und ist so bemessen, daß er vom Mittelfinger, Ringfinger und kleinem Finger der Hand einer Bedienungsperson umgriffen und gehalten werden kann.

Das innenliegende Ende des Einlaßstutzens 201 und das innenliegende Ene des Auslaßstutzens 202 münden beide versetzt gegenüberliegend in eine Ventilkammer 203, deren Mittelachse die Mittelachse der beiden Stutzen 201, 202 schneidet und sich senkrecht zu diesen erstreckt.

Die Ventilkammer 203 ist konisch ausgebildet und verjüngt sich von der Mündung des Einlaßstutzens 201 zum Eingang des Auslaßstutzens 202 hin. Sie erstreckt sich an ihrem verjüngten Ende nicht über den Auslaßstutzen 202 hinaus. Ihr anderes, sich verbreiterndes Ende reicht jedoch über die Einmündung des Einlaßstutzens 201 hinaus und geht dann in einen zylindrischen Abschnitt 204 über, dessen Länge etwa jener des sich verjüngenden, konischen Abschnitts entspricht.

Der zylindrische Abschnitt 204 geht an seinem von der Ventilkammer 203 abgewandten Ende in einen Gewindeabschnitt 205 mit einem Innengewinde über. Der kleinste Durchmesser des Innengewindes ist größer als jener des zylindrischen Abschnitts 204.

An das vom zylindrischen Abschnitt 204 abgewandte Ende des Gewindeabschnitts 205 schließt eine kreiszylindrische Aufnahme 206 an, deren Durchmesser wesentlich größer ist als jener des Gewindeabschnitts 205.

Die Länge der Ventilkammer 203, des zylindrischen Abschnitts 204, des Gewindeabschnitts 205 und der Aufnahme 206 sind abgestimmt auf die Abmessungen des Ventilkörpers 300. Die Maß-Zuordnung dieser Teilse ist in Fig. 5 ersichtlich.

Der Ventilkörper 300 ist als länglicher Stift mit einem konischen Endabschnitt 301 und einem sich daran anschließenden kreiszylindrischen Abschnitt 302 ausgebildet. Der Endabschnitt 301 ist komplementär konisch zur Ventilkammer 203 ausgebildet. Das dickere Ende des konischen Endabschnitts 301 geht bündig in den zylindrischen Abschnitt 302 über. Letzterer weist an seinem freien Ende einen Sechskant 303 auf.

Im zylindrischen Abschnitt 302 ist nahe dem konischen Abschnitt 301 eine Ringnut 304 zur Aufnahme des Dichtunsringes 500 angeordnet. In Wirkstellung liegt dessen Außenumfang dichtend an der Innenumfangswand des zylindrischen Abschnitts 204 des Ventilgehäuses 200 an.

Am kreiszylindrischen Abschnitt 302 befindet sich etwa in der Mitte zwischen der Ringnut 204 und dem sechskantigen Ende 303 ein Abschnitt 305 mit vergrößertem Außendurchmesser, der ein Außengewinde aufweist. Dieses Außengewinde ist passend zum Gewinde des Gewindeabschnitts 205 des Ventilgehäuses 200 ausgebildet. Die Gewindeabschnitte 205, 305 sind so positioniert, daß der konische Abschnitt 301 des Ventilkörpers 300 gerade dichtend an der Innenwand der Ventilkammer 203 anliegt, wenn die beiden Gewindeabschnitte 205, 305 voll ineinandergeschraubt sind, die beiden, der konischen Ventilkammer zugewandten Enden der Gewindeabschnitte 205, 305 also aneinanderschlagen. Es kann auch vorteilhaft sein, daß der konische Ventilkörper-Abschnitt 301 noch einen geringen kapillaren Abstand zur Ventilkammer-Innenwand aufweist, wenn die Gewindeabschnitte 205 und 305 voll ineinandergeschraubt sind. Hierdurch wird nämlich verhindert, daß bei zu tief eingefahrenem Ventilkörper 300 die Wand der Ventilkammer 203 gesprengt wird.

Über das zapfenförmige Ende 303 des Ventilkörpers 300 ist die Lagerbüchse 600 geschoben. Diese ist als Zylinderbüchse mit einem endseitigen Innenflansch ausgebildet. Dieser Innenflansch weist seineresits eine mittige Bohrung auf, die zwar groß genug ist, um das zapfenförmige Ende 303 des Ventilkörpers 300 durchzulassen, aber so bemessen ist, daß der Gewindeabschnitt 305 des Ventilkörpers 300 gegen den Innenflansch der Lagerbüchse 600 in seiner einen Endstellung anschlägt. Bevorzugt sind der Innendurchmesser der mittigen Bohrung des Innenflansches und der Außendurchmesser des zylindrischen Abschnittes 302 so aneinander angepaßt, daß sie im Bereich ihrer gegenseitigen Berührung während ihrer relativen koaxialen Verschiebung eine Keimsperre bilden. Hierzu genügt meistens ein Paßsitz der beiden Teile.

Die Lagerbüchse 600 ist an ihrer Außenfläche passend zur Aufnahme 206 ausgebildet und in diese eingeklebt.

Das Betätigungsorgan 400 ist im wesentlichen aus zwei konzentrisch zueinander angeordneten Büchsen, nämlich einer kreiszylindrischen Außenbüchse 406 und einer Innenbüchse 402, aufgebaut. Die Außenbüchse 406 weist eine außenseitige Rändelung 401 auf. Der Innendurchmesser der Außenbüchse 406 ist so bemessen, daß sie mit Spielpassung über den Außenumfang der Aufnahme 206 paßt.

Am einen Ende der Aufnahmebüchse 406 ist ein Innenflansch angeordnet, der die Innenbüchse 402 trägt. Letztere steht beidseitig vom Innenflansch vor und weist einen auf den Außensechskant 303 passenden Innensechskant 403 auf. Die Innenbüchse 402 weist einen Außendurchmesser auf, der in führenden Gleiteingriff mit dem Innendurchmesser der Lagerbüchse 600 treten kann. Nach Aufstecken des Innensechskants 403 auf den Außensechskant 303 des Ventilkörpers 300 ist also das Betätigungsorgan 400 für eine Drehbewegung geführt gelagert.

Das Material der Lagerbüchse 600 weist gegenüber jenem der Innenbüchse 402 einen nur geringen Reibungsbeiwert auf.

Im übrigen ist das Betätigungsorgan 400 aus Kunststoff geformt, der bevorzugt so eingefärbt ist, daß er beim flüchtigen Kontrollieren der Infusionseinrichtung eines Patienten ins Auge fällt, so daß dann, wenn versehentlich dieses Betätigungsorgan 400 am Dosierventil verblieben ist, dieser Umstand sofort entdeckt und abgestellt werden kann.

Das Ventilgehäuse 200 besteht bevorzugt aus einem klar-durchsichtigen, farblosen Material, etwa Acrylglas.

Der Dichtungsring 500 weist bevorzugt eine auffällige Färbung auf, so daß dessen Lage und somit auch die Position des Ventilkörpers 300 im Ventilgehäuse 200 ohne weiteres und auf einen Blick erkennbar ist.

Dem Dichtungsring 500 gegenüberliegend ist am Ventilgehäuse 200 eine Skala 207 zur Voreinstellung angeformt.

Der beim Einbau abwärts weisende Ablaßstutzen 202 kann nicht nur dazu dienen, während der Bedienung des aufgesteckten Betätigungsorgans 400 mit dem Daumen und dem Zeigefinger von den letzten drei Fingern der Bedienhand gehalten zu werden, sondern es ist auch möglich, diesen Auslaßstutzen 202 als Abstützeinrichtung zur Drehmomentabstützung eines Stellantriebs vorzusehen. In diesem Fall kann der Stellantrieb mit einem gabelartigen Element den Auslaßstutzen 202 beidseitig umgreifen, kann aber auch an abgesetzter Stelle ortsfest montiert sein und beispielsweise über eine biegsame Welle mit dem Ventilkörper 300 verbunden sein, wobei dann eine gesonderte Drehmomentabstützung etwa in Form eines am Auslaßstutzen 202 befestigten Gewichts vorgesehen ist, die am Dosierventil angreift. Schließlich ist es auch möglich, zusätzlich zu einem einen Handgriff bildenden Element oder anstelle dessen eine speziell zu einem Stellantrieb passend ausgebildete Drehmomentabstützung vorzusehen.

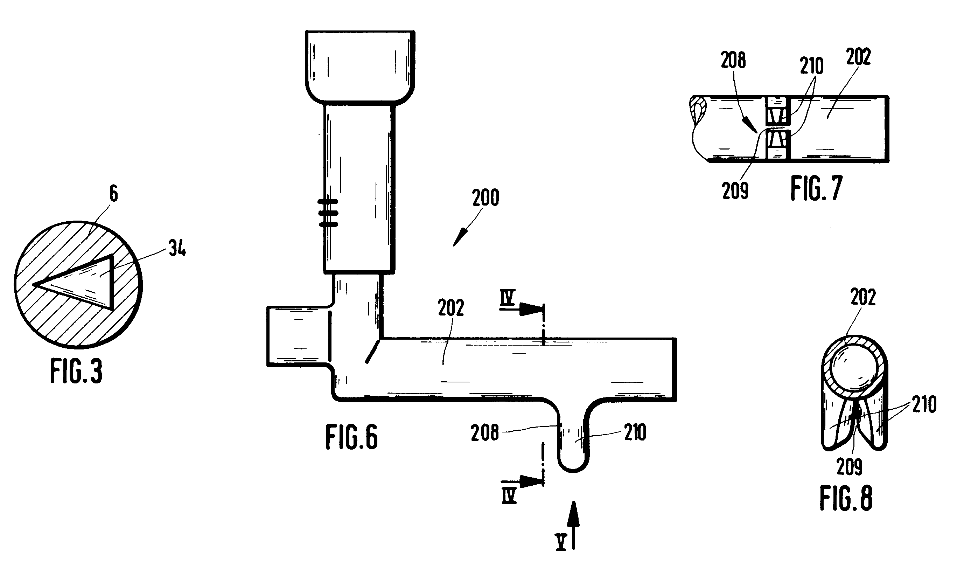

In Fig. 6 ist das Ventilgehäuse 200 einer anderen Ausführungsform des Dosierventils gezeigt: dieses Ventilgehäuse weist im Unterschied zu jenem der Ausführungsform der Fig. 4 und 5 zusätzlich am als Handgriff ausgebildeten Auslaßstutzen 202 auf dessen vom Betätigungsorgan 400 (in Fig. 6 nicht gezeigt) abgewandter Seite eine angeformte Schlauchklemme 208 auf, die, wie aus Fig. 7 besonders gut ersichtlich, aus zwei Klemschenkeln 210 gebildet ist, die sich beide parallel zueinander sowie senkrecht zum Auslaßstutzen 202 erstrecken und deren Außenseiten bündig in den Außenumfang des Auslaßstutzens 202 übergehen.

Zwischen den beiden Klemmschenkeln 210 ist ein Klemmschlitz 209 gebildet, dessen Breite so bemessen ist, daß ein in diesen eingeführter, am Ende des Auslaßstutzens 202 angeschlossener und zu einer Schleife gelegter Schlauch (nicht gezeigt) unter Bildung einer zuverlässigen Absperrung abgequetscht wird.

Die äußeren Enden der Klemmschenkel 210 sind abgerundet, um irgendeine Beschädigung des Schlauches zu vermeiden.

Wie besonders aus Fig. 8 ersichtlich ist, sind die Klemmschenkel 210 zum Klemmschlitz 209 hin abgeschrägt, wobei eine nur schmale Klemmstelle gebildet ist und dennoch das Einführen des Schlauches in den Klemmschlitz 209 erleichtert wird.

Ferner weisen, wie aus Fig. 7 ersichtlich, die freien Enden der Klemmschenkel 210 an ihren freien Enden zum Klemmschlitz 209 hin eine Abschrägung auf, die einerseits das Einführen des Schlauches in den Klemmschlitz erleichtert und andererseits einen Sitz für den Schlauch bietet. So ist es möglich, den Schlauch beispielsweise mittels Heftpflasters am Auslaßstutzen so zu befestigen, daß er durch die in Fig. 4 erkennbare Abschrägung läuft und an dieser ständig anliegt. Wenn nun rasch die Medikamentenzufuhr zum Patienten unterbrochen werden soll, dann genügt es, mit den Fingern oder der Handfläche der den Auslaßstutzen 202 umgreifenden Hand den Schlauch rasch in den Klemmschlitz 209 einzudrücken. Es kann somit beispielsweise die Absperrung der Medikamentenzufuhr sehr viel rascher vorgenommen werden als etwa durch Verstellen des Betätigungsorganes 400.

Zum Lösen des Schlauches aus dem Klemmschlitz genügt es, mit der Fingerspitze zwischen den Schlauch und die benachbarte Außenseite des Außenstutzens 202 zu greifen und hierbei den Schlauch aus dem Klemmschlitz 209 herauszuheben.

## Patentansprüche

1. Dosiervorrichtung zur Einstellung der Durchflußrate einer innerhalb eines Ventilgehäuses (2; 200) in einem Strömungskanal (8; 8'; 32; 33) in den oder aus dem Körper eines Lebewesens geführten Flüssigkeitsströmung, mit
a) einer Stelleinrichtung (1) zur Änderung des Strömungswiderstands des Strömrungskanals (8; 8'; 32; 33), und
b) einem Betätigungsorgan (4; 400) für die Stelleinrichtung (1),
dadurch **gekennzeichnet,** daß
c) eine Sicherungseinrichtung zum Schutz der Stelleinrichtung (1) gegen unabsichtliche oder absichtliche Verstellung vorgesehen ist,
d) die Sicherungseinrichtung im wesentlichen durch das Betätigungsorgan (4) und die Stelleinrichtung (1) gebildet ist und beide Teile hierzu sowie zu ihrem gegenseitigen lösbaren Eingriff nach Art eines Schlüssel-Schloß-Systems (11, 12) aneinander angepaßt sind,
e) das Betätigungsorgan (4) aus einer konzentrisch zu einer Innenbüchse (402) angeordneten kreiszylindrischen Außenbüchse (406) aufgebaut ist und mit dem Ventilgehäuse ein Handgriff (202) verbunden ist, der in einem solchen Abstand zur Außenbüchse (406) angeordnet ist, daß dessen feinfühlige und mühelose Bedienung mit dem Daumen und Zeigefinger einer Hand erfolgen kann, während deren drei übrige Finger den Handgriff umgreifen, ohne daß gleichzeitig versehentlich der gegenseitige Eingriff von Betätigungsorgan (4; 400) und Stelleinrichtung (1) gelöst wird.

2. Dosiereinrichtung nach Anspruch 1, dadurch gekennzeichnet, daß die Stelleinrichtung (1) einen im vom Strömungskanal (8; 8'; 32; 33) durchzogenen Ventilgehäuse zur Änderung des Strömungswiderstandes bewegbar geführten, nach Art eines Ventilkörpers ausgebildeten Körper (3; 300) aufweist, der an seinem dem Strömungskanal (8; 8'; 32; 33) abgewandten Endabschnitt (20') mit dem Betätigungsorgan (4; 400) in Eingriff bringbar ist.

3. Dosiervorrichtung nach Anspruch 2, dadurch gekennzeichnet, daß der Körper (3) über ein Gewinde (5) im Gehäuse (2; 200) geführt und das Betätigungsorgan als Drehknopf (4; 400) ausgebildet ist.

4. Dosiervorrichtung nach einem der Ansprüche 2 oder 3, dadurch gekennzeichnet, daß
a) der Körper (3; 300) und das Gehäuse (2; 200) koaxial ineinander angeordnet sind,
b) das Gehäuse (2; 200) mit den Enden des Strömungskanals (8; 8'; 32; 33) strömungsmäßig verbundene Anschlußstutzen (6, 7) aufweist,
c) die Achsen der Anschlußstutzen (6, 7) in Gehäuseachsrichtung gegeneinander versetzt sind, und
d) das Gehäuse (2; 200) und der Körper (3; 300) im Versatzbereich der Anschlußstutzen derart aneinander angebracht sind, daß
e) der Strömungswiderstand durch Änderung des Querschnittes und/oder der Länge des Strömungskanals (8; 8'; 32: 33) änderbar ist.

5. Dosiereinrichtung nach Anspruch 4, dadurch gekennzeichnet, daß das Gehäuse (2; 200) und der Körper (3; 300) im Versatzbereich der Anschlußstutzen (6, 7) zueinander komplementäre, den Strömungskanals-Querschnitt bestimmende Konusflächen (9, 10) aufweisen, deren gegenseitiger Abstand durch eine axiale Verschiebung des Körpers (3; 300) änderbar ist, und daß die Innenwandung (25) des Gehäuses (2; 200) und/oder die Außenwandung (26) des Körpers, (3; 300) im Versatzbereich des Anschlußstutzens (6, 7) gewindeartig ausgebildet sind/ist, derart, daß der Gewindegang (32; 33) als Strömungskanal wirkt, dessen Länge durch eine axiale Verschiebung des Körpers (3; 300) im Gehäuse (2; 200) änderbar ist.

6. Dosiervorrichtung nach Anspruch 1, dadurch gekennzeichnet, daß der Ventilkörper (300) stiftartig ausgebildet ist, ein Betätigungsmechanismus (205; 303, 305) vorgesehen ist, der ein am Ventilkörper (300) ausgebildetes Außengewinde (305) und ein hierzu komplementäres Innengewinde (205) am Ventilgehäuse (200) aufweist, ein Betätigungsanschluß (303) am Betätigungsmechanismus als ein um seine Längsachse drehbarer unrunder Zapfen (Sechskantzapfen 303) ausgebildet ist, und das Betätigungsorgan (400) eine zum Zapfen komplementäre durchgehende Bohrung (403) aufweist.

7. Dosierventil nach einem der Ansprüche 1 bis 6, gekennzeichnet durch eine vom Ventilkörper (300) unabhängige und diesem bevorzugt nachgeschaltete Absperreinrichtung, die als eine am Gehäuse (200) angeforamte Schlauchklemme (208) ausgebildet ist.

8. Dosierventil nach Anspruch 7, dadurch gekennzeichnet, daß die Schlauchklemme (208) aus zwei gabelartig einen Klemmschlitz (209) einschließenden Klemmschenkeln (210) gebildet ist, die an der Aussenseite des Handgriffs (202) angeformt sind.

## Claims

1. A metering device for setting the flow rate of a flow of liquid conveyed inside a valve housing (2; 200) in a flow channel (8; 8'; 32; 33) into or out of the body of a living being, having
a) an adjusting device (1) for changing the flow resistance of the flow channel (8; 8'; 32; 33), and
b) an operating element (4; 400) for the adjusting device (1),
characterised in that
c) a safety device is provided for protecting the adjusting device (1) from unintentional or intentional readjustment,
d) the safety device is substantially formed by the operating element (4) and the adjusting device (1), and both parts are adapted to each other in the manner of a key locking system (11, 12) both for this purpose and for their mutual releasable engagement,
e) the operating element (4) is constructed from a circular cylindrical outer sleeve (406) arranged concentrically to an inner sleeve (402), and to the valve housing is connected a handle (202) which is arranged at a distance from the outer sleeve (406) such that it can be manipulated sensitively and effortlessly using the thumb and forefinger of one hand, while the three remaining fingers grip the handle without the mutual engagement of operating element (4; 400) and adjusting device (1) being simultaneously inadvertently released.

2. A metering device according to claim 1, characterised in that the adjusting device (1) has a body (3; 300) which in order to change the flow resistance is movably guided in the valve housing through which the flow channel (8; 8'; 32; 33) passes, is formed in the manner of a valve body and can be brought into engagement with the operating element (4; 400) on its end portion (20' ) facing away from the flow channel (8; 8'; 32; 33).

3. A metering device according to claim 2, characterised in that the body (3) is guided in the housing (2; 200) via a thread (5) and the operating element is formed as a rotary knob (4; 400).

4. A metering device according to either one of claims 2 and 3, characterised in that
a) the body (3; 300) and the housing (2; 200) are arranged coaxially one inside the other,
b) the housing (2; 200) has connecting branches (6, 7) connected to the ends of the flow channel (8; 8'; 32; 33) according to the flow,
c) the axes of the connecting branches (6, 7) are staggered in the axial direction of the housing, and
d) the housing (2; 200) and the body (3; 300) are mounted on each other in the offset region of the connecting branches in such a way that,
e) the flow resistance can be changed by changing the cross-section and/or the length of the flow channel (8; 8'; 32; 33).

5. A metering device according to claim 4, characterised in that the housing (2; 200) and the body (3; 300) have, in the offset region of the connecting branches (6, 7), tapered surfaces (9, 10) which are complementary to each other and determine the flow channel cross-section and the mutual distance of which can be changed by axial displacement of the body (3; 300), and in that the inner wall (25) of the housing (2; 200) and/or the outer wall (26) of the body (3; 300) is/are formed in the manner of a thread in the offset region of the connecting branch (6, 7) in such a way that the thread (32; 33) acts as a flow channel, the length of which can be changed by axial displacement of the body (3; 300) in the housing (2; 200).

6. A metering device according to claim 1, characterised in that the valve body (300) is formed in the manner of a pin, an operating mechanism (205; 303, 305) is provided which has an outer thread (305) formed on the valve body (300) and a complementary inner thread (205) on the valve housing (200), an operating connection (303) on the operating mechanism is formed as a non-circular pivot (hexagonal pivot 303) rotatable about its longitudinal axis, and the operating element (400) has a through bore (403) complementary to the pivot.

7. A metering valve according to any one of claims 1 to 6, characterised by a shut-off device which is independent of the valve body (300) and preferably connected downstream thereof and which is formed as a tube clamp (208) formed on the housing (200).

8. A metering valve according to claim 7, characterised in that the tube clamp (208) is formed from two clamping arms (210) enclosing a clamping slot (209) in a fork-like manner and formed on the outside of the handle (202).

## Revendications

1. Dispositif de dosage pour le réglage du débit d'un courant de liquide circulant dans ou hors du corps d'un être vivant dans un canal d'écoulement (8, 8'; 32, 33) d'un boîtier de valve (2; 200) avec:
a) un dispositif de positionnement (1) pour la modification de la résistance à l'écoulement du canal d'écoulement (8, 8'; 32, 33), et
b) un organe d'actionnement (4; 400) de ce dispositif de positionnement (1);
caractérisé en ce que:
c) il est prévu un dispositif de sécurité pour la protection du dispositif de réglage (1) contre les défauts de mise au point volontaires ou involontaires,
d) le dispositif de sécurité est essentiellement constitué par l'organe d'actionnement (4) et le dispositif de réglage (1) et ces deux éléments sont adaptés l'un à l'autre pour coopérer mutuellement de manière amovible à la façon d'un système clé-serrure (11, 12),
e) l'organe d'actionnement (4) est constitué d'un manchon extérieur cylindrique (406) disposé de manière concentrique à un manchon intérieur (402) et avec le boîtier de valve est liée une poignée (202) qui est disposée à une distance telle du manchon extérieur (406) que la manipulation de celle-ci peut être effectuée de manière fine et sans peine avec le pouce et de l'index d'une main tandis que les trois autres doigts de cette main saisissent la poignée sans que simultanément, par inadvertance, la coopération mutuelle de l'organe d'actionnement (4; 400) et de l'organe de réglage (1) puisse être détruite.

2. Dispositif de dosage selon la revendication 1, caractérisé en ce que le dispositif de réglage comporte un corps (3; 300) réalisé à la manière d'un corps de valve aidé de manière mobile dans le boîtier de valve traversé par le canal d'écoulement (8, 8'; 32, 33) pour modifier la résistance à l'écoulement, ledit corps pouvant être mis en prise par sa section terminale (20') opposée au canal d'écoulement (8, 8'; 32, 33) avec l'organe d'actionnement (4; 400).

3. Dispositif de dosage selon la revendication 2, caractérisé en ce que le corps (3) est guidé au moyen d'un filetage (5) du boîtier (2; 200) et en ce que l'organe d'actionnement a la forme d'un bouton rotatif (4; 400).

4. Dispositif de dosage selon l'une des revendications 2 ou 3, caractérisé en ce que:
a) le corps (3; 300) et le boîtier (2; 200) sont disposés coaxialement l'un à l'autre;
b) le boîtier (2; 200) comporte des ajutages de raccordement (6, 7) liés pour permettre l'écoulement aux extrémités du canal d'écoulement (8, 8'; 32, 33);
c) les axes des ajutages de raccordement (6, 7) sont décalés l'un par rapport à l'autre selon la direction de l'axe du boîtier; et
d) le boîtier (2; 200) et le corps (3; 300) sont disposés l'un par rapport à l'autre dans la zone de chevauchement des ajutages de raccordement pour que:
e) la résistance à l'écoulement soit modifiable par variation de la section et/ou de la longueur du canal d'écoulement (8, 8'; 32, 33).

5. Dispositif de dosage selon la revendication 4, caractérisé en ce que le boîtier (2; 200) et le corps (3; 300) dans la zone de chevauchement des ajutages de raccordement (6, 7) comporte des surfaces coniques (9, 10) complémentaires l'une de l'autre et déterminant la section du canal d'écoulement dont la distance mutuelle est modifiable par un déplacement axial du corps (3; 300) et en ce que la paroi intérieure (25) du boîtier (2; 200) et/ou la paroi extérieure (26) du corps (3; 300) est conformée en hélice dans la zone de chevauchement des ajutages de raccordement (6, 7) de telle sorte que le parcours hélicoïdal (32, 33) agisse comme canal d'écoulement dont la longueur est modifiable par un déplacement axial du corps (3, 300) dans le boîtier (2, 200).

6. Dispositif de dosage selon la revendication 1, caractérisé en ce que le corps de valve (300) a la forme d'une branche, un mécanisme d'actionnement (205, 303, 305) est prévu qui comporte un filetage extérieur ménagé dans le corps de valve (300) et un filetage intérieur complémentaire de celui-ci dans le boîtier de valve (200), un connecteur d'actionnement (303) pour mécanisme d'actionnement conformé une broche non circulaire, en particulier broche à six pans (303) mobile en rotation autour de son axe longitudinal et en ce que l'organe d'actionnement (400) est traversé par un alésage complémentaire de cette branche.

7. Dispositif de dosage selon l'une des revendications 1 à 6, caractérisé en un dispositif de blocage indépendant du corps de valve (300) et en aval de celui-ci, dispositif formé par un dispositif de pincement (208) du tube ménagé sur le boîtier (200).

8. Dispositif de dosage selon la revendication 7, caractérisé en ce que le dispositif de pincement de tube (208) est formé de deux doigts de serrage (210) entourant à la manière d'une fourche une fente de serrage (209), lesquels sont ménagés sur le côté extérieur de la poignée (202).
